# EUROPÄISCHE PATENTANMELDUNG

(11) **EP 4 368 603 A1**
(43) Veröffentlichungstag der Anmeldung: **15.05.2024**
(21) Anmeldenummer: 22206313.3
(22) Anmeldetag: 09.11.2022
(51) Int. Cl.: C07C 1/20, C07C 7/04, C07C 11/04, C07C 11/06, C07C 15/04

(54) **VERFAHREN ZUR HERSTELLUNG VON OLEFINEN AUS OXYGENATEN MIT VARIABLER PRODUKTION AN ETHYLEN UND PROPYLEN**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: HAAG, Stephane, 60388 Frankfurt am Main (DE); GORNY, Martin, 60439 Frankfurt am Main (DE); WILLIAMS, Bryce, 60388 Frankfurt am Main (DE); RENNER, Thomas, 60388 Frankfurt am Main (DE)

(57) **Zusammenfassung**

Die Erfindung betrifft ein Verfahren zur Herstellung von Olefinen aus Oxygenaten mit variabler Produktion an Ethylen und Propylen. Erfindungsgemäß erfolgt eine Umstellung des Verfahrens ausgehend von einem Zustand 1 mit niedrigerer Ethylenproduktion hin zu einem Zustand 2 mit höherer Ethylenproduktion dadurch, dass:
- der Anteil C2re des Ethylenproduktstroms, der zum OTO-Reaktor zurückgeführt wird, von einem ersten Wert C2re,1 auf einen zweiten Wert C2re,2 abgesenkt wird, wodurch sich eine Änderung dC2re = C2re,1 - C2re,2 ergibt,
- der Anteil C4+re des C4+ Olefine enthaltenden C4+ Produktstroms, der zum OTO-Reaktor zurückgeführt wird, von einem ersten Wert C4+re, 1 auf einen zweiten Wert C4+re,2 erhöht wird, wodurch sich eine Änderung dC4+re = C4+re,2 - C4+re,1 ergibt,
- wobei die Änderungen dC2re und dC4+re in einem Massenstromverhältnis dC4+re / dC2re stehen, wobei das Massenstromverhältnis zwischen 70 % und 130 %, bevorzugt zwischen 80 % und 120 %, weiter bevorzugt zwischen 90 % und 110 %, meist bevorzugt 100% beträgt.

## Beschreibung

### Gebiet der Erfindung

Die Erfindung betrifft ein Verfahren zur Herstellung von Olefinen aus Oxygenaten (OTO) mit variabler Produktion an Ethylen und Propylen. Insbesondere betrifft die Erfindung eine Ausgestaltung eines spezifischen OTO-Verfahrens, die es gestattet, Ethylen und Propylen mit variablen Anteilen am Gesamtprodukt herzustellen, um diese kurzkettigen Olefine als Monomere in einer nachgeschalteten Polymerisation, beispielsweise einer Copolymerisation von Ethylen und Propylen, insbesondere einer Erzeugung von Polypropylen (PP) mit Ethylen als Copolymer-Anteil, als Einsatzstoff einzusetzen.

### Stand der Technik

Kurzkettige Olefine, beispielsweise Propylen (Propen) und Ethylen (Ethen), gehören zu den wichtigsten Grundstoffen der chemischen Industrie. Dies liegt darin begründet, das ausgehend von diesen ungesättigten Verbindungen mit einer kurzen Kettenlänge Moleküle mit langkettigem Kohlenstoffgerüst und zusätzlichen Funktionalisierungen aufgebaut werden können. Insbesondere bei der Erzeugung von Kunststoffen durch Polymerisation finden diese kurzkettigen Olefine weite Anwendung.

Als Quelle für kurzkettige Olefine diente in der Vergangenheit vor allem das Steam-Cracking, d. h. die thermische Spaltung bei der Erdölverarbeitung. In den vergangenen Jahren wurden jedoch weitere Prozesse zur Herstellung von kurzkettigen Olefinen entwickelt. Dies ist zum einen durch steigende Nachfrage bedingt, die durch die vorhandenen Quellen nicht mehr gedeckt werden kann; zum anderen fordert die zunehmende Verknappung von fossilen Rohstoffen die Verwendung anderer Ausgangsstoffe.

Die sog. MTP- (Methanol-to-Propylen) oder auch MTO- (Methanol-to-Olefin) Verfahren zur Herstellung von Propylen und anderen kurzkettigen Olefinen gehen von Methanol als Ausgangsstoff aus. Verallgemeinernd spricht man in diesem Zusammenhang auch von Oxygenat-zu-Olefin-(OTO)-Verfahren, da sauerstoffhaltige organische Komponenten wie Methanol oder Dimethylether (DME) auch als Oxygenate bezeichnet werden. In diesen heterogen katalysierten Verfahren wird demnach beispielsweise aus Methanol zunächst teilweise das Zwischenprodukt Dimethylether und nachfolgend aus einer Mischung von Methanol und Dimethylether eine Mischung aus Ethylen und Propylen sowie Kohlenwasserstoffen mit höherer Molmasse, darunter auch Olefine, gebildet. Zudem findet sich im Produktstrom Wasser, welches einerseits einen aus dem Prozessdampf stammt, der optional dem MTO-Reaktor zur Reaktionsmodulierung zugeführt wird und andererseits aus dem im MTP-Reaktor erzeugten Reaktionswasser, das als Koppelprodukt der Olefinbildungsreaktion entsteht.

Die sich anschließende Aufreinigung soll zum einen unerwünschte Nebenprodukte und unumgesetzte Edukte abtrennen und die einzelnen Kohlenwasserstofffraktionen möglichst rein darstellen. Üblicherweise wird hierzu im ersten Schritt ein Quenchsystem zur Anwendung gebracht. Unter Quenchen wird dabei eine schlagartige oder schockartige Abkühlung verstanden, die zumeist durch direkten Wärmeaustausch mit einem fluiden Quenchmedium bewirkt wird. Wenn hierzu eine Flüssigkeit wie Wasser oder Methanol verwendet wird, tritt zudem eine gewisse Reinigungswirkung in Bezug auf die verbliebene Gasphase auf.

Ein Beispiel für die einer OTO-Reaktion nachfolgende Aufreinigung findet sich in der Patentveröffentlichung DE 10 2014 112 792 A1, in der beschrieben wird, wie in einem ersten Schritt eine heterogen-katalysierte Umsetzung von wenigstens einem Oxygenat zu einem C2 Olefine, C3 Olefine, C4 Olefine, C5/6 Kohlenwasserstoffverbindungen und C7+ Kohlenwasserstoffverbindungen enthaltenden Produktstrom und in einem zweiten Schritt eine Abtrennung eines zu wenigstens 95 Gew.-% aus C3 Olefinen bestehenden Propylenstroms erzeugt wird.

Die weiteren Aufreinigungseinheiten, die in der DE 10 2014 112 792 A1 beschrieben werden, entsprechen dem im Stand der Technik üblichen Konzept. Durch das Quenchen kann bereits eine grobe Trennung der Fraktionen in Abhängigkeit von ihrer Kettenlänge der entstehenden Olefine aufgrund des teilweisen Auskondensierens erfolgen, so dass eine flüssige C4+ Fraktion aus dem Quench abgeführt werden kann. Die gasförmig abgetrennte C4- Fraktion wird anschließend in eine Kompressionsstufe eingespeist. Die aus der Kompression stammende C4- Fraktion wird dann einer Trennvorrichtung zugeführt, in der C3- Kohlenwasserstoffe von den C4+ Kohlenwasserstoffen separiert werden. In anschließenden Reinigungsschritten wird die C3 Fraktion in einer weiteren Trenneinrichtung von der C2- Fraktion getrennt, was aufgrund der geringen Siedepunkte der beiden Fraktionen unter Druck erfolgen muss. Insgesamt ist die Aufreinigung des Produktstroms kompliziert, da eine hohe Reinheit der Produkte angestrebt wird. Dies gilt für MTP-Anlagen mit üblichen Produktionskapazitäten von etwa 470 kta Propylen, wird aber noch entscheidender, wenn die Anlagenkapazität geringer ist (100 kta oder 200 kta Propylen). Dadurch sind kleine Anlagen wirtschaftlich weniger rentabel.

Insgesamt umfassen die Produkte des MTP-Verfahrens einen Propylenproduktstrom, einen Ethylenproduktstrom und eine Benzinfraktion, wobei letztere höhere Paraffine, Olefine, Aromaten und cyclische Kohlenwasserstoffe enthält. Ferner werden innerhalb des Verfahrens verschiedene Kohlenwasserstoffe, vor allem C4 Olefine, C5 Olefine und C6+ Olefine enthaltende Ströme gewonnen, die überwiegend oder vollständig zum MTP-Reaktor zurückgeführt werden und dort katalytisch zu kurzkettigen Olefinen gespaltet werden, was die Ausbeute der Zielprodukte Propylen und Ethylen verbessert.

Das MTP-Verfahren zielt in seiner bisherigen Ausgestaltung vor allem auf die Maximierung der Ausbeute an Propylen ab, das ein besonders begehrtes Produkt darstellt. Es besteht allerdings auch das Bedürfnis, Ethylen und Propylen, idealerweise mit variierbaren Anteilen, für eine Copolymerisation der beiden Olefine zur Verfügung zu stellen. Hierfür bieten die aus dem Stand der Technik bekannten Ausgestaltungen des MTP-Verfahrens und anderer OTO-Verfahren bislang keine befriedigende Lösung an.

### Beschreibung der Erfindung

Es ist daher die Aufgabe der vorliegenden Erfindung, ein Verfahren zur Herstellung von Olefinen aus Oxygenaten mit variabler Produktion an Ethylen und Propylen vorzuschlagen, das die genannten Nachteile von OTO-Verfahren vermeidet, die bislang aus dem Stand der Technik bekannt sind. Diese Aufgabe wird durch ein Verfahren mit den Merkmalen des Anspruchs 1 gelöst.

Unter Oxygenaten werden grundsätzlich alle sauerstoffhaltigen Kohlenwasserstoffverbindungen verstanden, die sich unter Oxygenatumwandlungsbedingungen zu Olefinen, insbesondere zu kurzkettigen Olefinen wie Propylen, und weiteren Kohlenwasserstoffprodukten umsetzen lassen.

Die für die Umsetzung von Oxygenaten zu Olefinprodukten erforderlichen Oxygenatumwandlungsbedingungen sind dem Fachmann aus dem Stand der Technik, beispielsweise den eingangs erörterten Druckschriften, bekannt. Es sind diejenigen physikalisch-chemischen Bedingungen, unter denen ein messbarer, bevorzugt ein technisch relevanter Umsatz von Oxygenaten zu Olefinen erzielt wird. Notwendige Anpassungen dieser Bedingungen an die jeweiligen Betriebserfordernisse wird der Fachmann auf der Grundlage von Routineversuchen vornehmen. Dabei können ggf. offenbarte, spezifische Reaktionsbedingungen als Orientierung dienen, sie sind aber in Bezug auf den Umfang der Erfindung nicht einschränkend zu verstehen.

Thermische Trennverfahren im Sinne der vorliegenden Erfindung sind alle Trennverfahren, die auf der Einstellung eines thermodynamischen Phasengleichgewichtes beruhen. Bevorzugt sind dies die Destillation oder Rektifikation. Grundsätzlich ist aber auch der Einsatz anderer thermischer Trennverfahren denkbar, beispielsweise der Extraktion oder Extraktivdestillation.

Unter einem Verfahren zur Herstellung von Olefinen mit variabler Produktion an Ethylen und Propylen wird verstanden, dass das Massenverhältnis der Zielprodukte Ethylen und Propylen durch die erfindungsgemäßen Änderungen der Verfahrensbedingungen in beabsichtigter und reproduzierbarer Weise verändert wird. Die Adjektive "variabel" und "variierbar" sind dabei für die Zwecke der vorliegenden Offenbarung als Synonyme zu verstehen.

Die Unterscheidung zwischen einem ersten Zustand 1 mit niedrigerer Ethylenproduktion und einem zweiten Zustand 2 mit höherer Ethylenproduktion ist rein qualitativ zu verstehen und bezogen auf relative Änderungen des als Zielprodukt aus dem Verfahren ausgeleiteten Ethylen-Massenstroms.

Die Angabe, dass das Absenken des Anteils C2re und das Erhöhen mindestens eines Anteils, ausgewählt aus der Gruppe, umfassend: C4+re, C4re, C5re, C6+re, nachfolgend rückgängig gemacht werden, um das Verfahren von einem zweiten Zustand 2 mit höherer Ethylenproduktion zurück in einen ersten Zustand 1 mit niedrigerer Ethylenproduktion zu überführen, ist so zu verstehen, dass zunächst eine Änderung der Verfahrensdurchführung im Sinne des Anspruchs 1 vom Zustand 1 in den Zustand 2 vorgenommen wurde und das Verfahren anschließend durch vollständige oder teilweise Rücknahme der Änderungen wieder vom Zustand 2 in den Zustand 1 verbracht wird.

Unter der Angabe, dass ein stationärer Betrieb des Gesamtverfahrens und der einzelnen Verfahrensstufen gewährleistet ist, ist bezogen auf das Gesamtverfahren zu verstehen, dass für einen definierten Satz von Verfahrensbedingungen innerhalb gewisser Zeit ein Zustand erreicht wird, bei dem über eine längere Zeitdauer oder Betriebsdauer des erfindungsgemäßen Verfahrens konstante Massenströme der Zielprodukte Ethylen und Propylen aus dem Verfahren ausgeleitet werden. Auf einzelne Verfahrensstufen bezogen ist dies ebenfalls so zu verstehen, dass für die betrachtete Verfahrensstufe ein stationärer Zustand erreicht wird und beispielsweise eine Überfüllung und ein Leerlaufen von Trennkolonnen vermieden wird. Die Betriebsdauer ist dabei die Zeitdauer, in der das Verfahren betrieben wird und dabei die Verfahrensprodukte erzeugt. Eine übliche Einheit hierfür ist Betriebsstunden h bzw. hos ("hours on stream").

Wenn angegeben wird, dass ein Strom Kohlenwasserstoffe umfasst und spezifisch Olefine umfasst, ist dies so zu verstehen, dass die Olefine eine spezifische Untergruppe der Kohlenwasserstoffe darstellen und dass demnach auch andere, nicht-olefinische Kohlenwasserstoffe in dem Strom enthalten sein können.

Als kurzkettige Olefine werden im Rahmen der vorliegenden Erfindung insbesondere die Olefine verstanden, die bei Umgebungsbedingungen gasförmig vorliegen, beispielsweise Ethylen, Propylen sowie die isomeren Butene 1-Buten, cis-2-Buten, trans-2-Buten, iso-Buten.

Als höhere Kohlenwasserstoffe werden im Rahmen der vorliegenden Erfindung insbesondere diejenigen Kohlenwasserstoffe verstanden, die bei Umgebungsbedingungen flüssig vorliegen.

Zur Bezeichnung von Kohlenwasserstofffraktionen wird folgende Nomenklatur verwendet: "Cn Fraktion" bezeichnet eine Kohlenwasserstofffraktion, die überwiegend Kohlenwasserstoffe der C-Kettenlänge n, also mit n C-Atomen, enthält. "Cn-Fraktion" bezeichnet eine Kohlenwasserstofffraktion, die überwiegend Kohlenwasserstoffe der C-Kettenlänge n sowie mit niedrigeren C-Kettenlängen enthält. "Cn+ Fraktion" bezeichnet eine Kohlenwasserstofffraktion, die überwiegend Kohlenwasserstoffe der C-Kettenlänge n sowie mit höheren C-Kettenlängen enthält. Aufgrund der verwendeten physikalischen Trennverfahren, beispielsweise der Destillation, ist die Auftrennung hinsichtlich der C-Kettenlänge nicht in der Weise zu verstehen, dass Kohlenwasserstoffe mit anderer Kettenlänge rigoros ausgeschlossen werden. So enthält eine Cn- Fraktion je nach Verfahrensbedingungen des Trennverfahrens immer noch geringe Mengen von Kohlenwasserstoffen mit einer C-Zahl größer als n.

Die genannten Aggregatzustände fest, flüssig und gasförmig bzw. dampfförmig sind immer in Bezug auf die lokalen physikalischen Bedingungen zu verstehen, die bei dem jeweiligen Verfahrensschritt oder in dem jeweiligen Anlagenteil herrschen, sofern nichts anderes angegeben ist. Im Rahmen der vorliegende Anmeldung sind die Aggregatzustände gasförmig bzw. dampfförmig als synonym zu betrachten.

Unter einem Aufteilen oder Auftrennen bzw. Abtrennen eines Stoffstroms ist im Zusammenhang mit der vorliegenden Erfindung die Erzeugung mindestens zweier Teilströme aus dem ursprünglichen Stoffstrom zu verstehen, wobei mit dem Auftrennen bzw. Abtrennen eine beabsichtigte Änderung der stofflichen Zusammensetzung der erhaltenen Teilströme in Bezug auf den ursprünglichen Stoffstrom verbunden ist, beispielsweise durch Anwendung eines thermischen Trennverfahrens oder zumindest thermischen Trennschrittes auf den ursprünglichen Stoffstrom. Dagegen ist mit dem Aufteilen des ursprünglichen Stoffstroms in der Regel keine Änderung der stofflichen Zusammensetzung der erhaltenen Teilströme verbunden.

Unter einer Benzinfraktion ist ein überwiegend, bevorzugt weitgehend vollständig aus höheren Kohlenwasserstoffen bestehendes, unter Umgebungsbedingungen flüssig vorliegendes Stoffgemisch zu verstehen, das geeignet sein kann, als Ottokraftstoff verwendet zu werden. Ein entsprechender Stoffstrom wird als Benzinproduktstrom bezeichnet.

Unter dem überwiegenden Teil einer Fraktion, eines Stoffstroms etc. ist ein Anteil zu verstehen, der mengenmäßig größer ist als alle anderen jeweils für sich betrachteten Anteile. Insbesondere bei binären Gemischen oder bei der Auftrennung einer Fraktion in zwei Teile ist darunter ein Anteil von mehr als 50 Gew.-% zu verstehen, sofern im konkreten Fall nichts anderes angegeben ist.

Unter der Angabe, dass ein Stoffstrom überwiegend aus einer Komponente oder Komponentengruppe besteht, ist zu verstehen, dass der Stoffmengenanteil (Molenbruch) oder Massenanteil (Massenbruch) dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Massenanteil (Massenbruch) zugrunde gelegt.

Unter der Ethylenproduktion bzw. der Propylenproduktion wird die pro Zeiteinheit erzeugte Menge an Ethylen bzw. Propylen verstanden. Eine übliche Einheit ist Tagestonnen (t/d).

Erfindungsgemäß erfolgt die Umstellung des Verfahrens ausgehend von einem Zustand 1 mit niedrigerer Ethylenproduktion hin zu einem Zustand 2 mit höherer Ethylenproduktion dadurch, dass:
- der Anteil C2re des Ethylenproduktstroms, der zum OTO-Reaktor zurückgeführt wird, von einem ersten Wert C2re,1 auf einen zweiten Wert C2re,2 abgesenkt wird, wodurch sich eine Änderung dC2re = C2re,1 - C2re,2 ergibt,
- der Anteil C4+re des C4+ Olefine enthaltenden C4+ Produktstroms, der zum OTO-Reaktor zurückgeführt wird, von einem ersten Wert C4+re, 1 auf einen zweiten Wert C4+re,2 erhöht wird, wodurch sich eine Änderung dC4+re = C4+re,2 - C4+re,1 ergibt,
- wobei die Änderungen dC2re und dC4+re in einem Massenstromverhältnis dC4+re / dC2re stehen, wobei das Massenstromverhältnis zwischen 70 % und 130 %, bevorzugt zwischen 80 % und 120 %, weiter bevorzugt zwischen 90 % und 110 %, meist bevorzugt 100% beträgt.

Überraschenderweise führt die erfindungsgemäße Umstellung der Verfahrensdurchführung vom Zustand 1 mit niedrigerer Ethylenproduktion hin zu einem Zustand 2 mit höherer Ethylenproduktion nicht zu einem Verlust des wertvollen Zielproduktes Propylen, sondern es nimmt die Ausbeute für das C4+ Paraffine und Aromaten enthaltende Benzinprodukt ab, das einen deutlich geringeren Wert aufweist.

Insgesamt lassen sich die mit der Erfindung verbundenen Vorteile wie folgt zusammenfassen:
- Die Produktion der wertvollen leichten, kurzkettigen Olefine, insbesondere des Ethylens und Propylens, wird erhöht.
- Es können mit dem Verfahren Produktströme mit verschiedenem Ethylen- und Propylengehalt als Monomere für eine nachfolgende Copolymerisation zur Verfügung gestellt werden.
- Die Befüllung etwa benötigter Ethylen-Lagertanks wird besser kontrolliert und besser an die Bedürfnisse einer etwa vorhandenen, nachgeschalteten Copolymerisation angepasst.
- Es resultiert eine zeitlich stabilere Verfahrensdurchführung.

### Weitere Aspekte der Erfindung

Ein zweiter Aspekt der Erfindung ist dadurch gekennzeichnet, dass sich beim Überführen des Verfahrens von einem ersten Zustand 1 mit niedrigerer Ethylenproduktion in einen zweiten Zustand 2 mit höherer Ethylenproduktion die Propylenproduktion sich nur um maximal 5 %, bevorzugt nur um maximal 3 % verringert. Die erzielten Werte für die Ethylenproduktion und die Propylenproduktion sind abhängig vom Konditionierungszustand des OTO-Katalysators, die beispielsweise bei 1000 Betriebsstunden abgeschlossen ist. Es resultiert dann ein als "Middle of Run" (MOR) bezeichneter Betriebszeitraum, in dem im Zustand 2 eine konstant hohe oder nur um maximal 5 %, bevorzugt nur um maximal 3 % verringerte Propylenproduktion bei gleichzeitig gegenüber Zustand 1 erhöhter Ethylenproduktion beobachtet wird.

Ein dritter Aspekt der Erfindung ist dadurch gekennzeichnet, dass sich beim Überführen des Verfahrens von einem ersten Zustand 1 mit niedrigerer Ethylenproduktion in einen zweiten Zustand 2 mit höherer Ethylenproduktion die Summe (Ethylenproduktion + Propylenproduktion) um mindestens 8 %, bevorzugt um mindestens 10 % erhöht. Die erzielten Werte für die Ethylenproduktion und die Propylenproduktion sind abhängig vom Konditionierungszustand des OTO-Katalysators, die beispielsweise bei 1000 Betriebsstunden abgeschlossen ist. Es resultiert dann ein als "Middle of Run" (MOR) bezeichneter Betriebszeitraum, in dem im Zustand 2 die Summe (Ethylenproduktion + Propylenproduktion) um mindestens 8 %, bevorzugt um mindestens 10 % erhöht werden kann. Ein geringfügiges Absinken der Propylenproduktion wird dabei durch die Steigerung der Ethylenproduktion überkompensiert. In einem Beispiel verringert sich die Propylenproduktion dabei nur um maximal 5 %, bevorzugt nur um maximal 3 %.

Ein vierter Aspekt der Erfindung ist dadurch gekennzeichnet, dass das Überführen des Verfahrens von einem ersten Zustand 1 mit niedrigerer Ethylenproduktion in einen zweiten Zustand 2 mit höherer Ethylenproduktion frühestens nach 600 Betriebsstunden, bevorzugt frühestens nach 800 Betriebsstunden, meist bevorzugt frühestens nach 1000 Betriebsstunden des OTO-Katalysators erfolgt. Untersuchungen zeigen, dass ab den genannten Zeitdauern nicht mehr mit einer signifikanten Abnahme der Propylenproduktion zu rechnen ist, wenn das Verfahren vom Zustand 1 in den Zustand 2 überführt wird. Ab den genannten Zeitdauern wird im Zustand 2 eine konstant hohe, dem Zustand 1 entsprechende Propylenproduktion bei gleichzeitig gegenüber Zustand 1 erhöhter Ethylenproduktion beobachtet.

Ein fünfter Aspekt der Erfindung ist dadurch gekennzeichnet, dass das Überführen des Verfahrens von einem ersten Zustand 1 mit niedrigerer Ethylenproduktion in einen zweiten Zustand 2 mit höherer Ethylenproduktion spätestens nach 200 Betriebsstunden, bevorzugt spätestens nach 100 Betriebsstunden, meist bevorzugt spätestens nach 50 Betriebsstunden des OTO-Katalysators erfolgt. Bei diesem Aspekt der Erfindung verringert sich zwar hierdurch temporär die Propylenproduktion, allerdings verkürzt sich hierdurch die Formierungsphase oder Konditionierungsphase, also derjenige Zeitraum, nach dem ein konstantes Reaktionsverhalten des OTO-Katalysators beobachtet wird ("Middle of Run", MOR), bevor - bei sehr langen Betriebszeiten im Betriebszeitraum "End of Run" (EOR) bei typischerweise mehr als 7000 hos - Alterungseffekte des OTO-Katalysators auftreten. Ab dem Betriebszeitraum "Middle of Run" (MOR) wird im Zustand 2 eine konstant hohe, dem Zustand 1 entsprechende Propylenproduktion bei gleichzeitig gegenüber Zustand 1 erhöhter Ethylenproduktion beobachtet.

Ein sechster Aspekt der Erfindung ist dadurch gekennzeichnet, dass der C4+ Olefine enthaltende C4+ Produktstrom mittels thermischer Trennverfahren weiter aufgetrennt wird in mindestens einen weiteren Olefinproduktstrom, ausgewählt aus einer Gruppe, die umfasst:
(c31) einen C4 Olefinproduktstrom, enthaltend Butene, der mindestens teilweise mit einem Anteil C4re zum OTO-Reaktor zurückgeführt wird, wobei der rückgeführte Anteil C4re von einem ersten Wert C4re,1 auf einen zweiten Wert C4re,2 erhöht wird, wodurch sich eine Änderung dC4re = C4re,2 - C4re,1 ergibt,
(c32) einen C5 Olefinproduktstrom, enthaltend Pentene, der mindestens teilweise mit einem Anteil C5re zum OTO-Reaktor zurückgeführt wird, wobei der rückgeführte Anteil C5re von einem ersten Wert C5re,1 auf einen zweiten Wert C5re,2 erhöht wird, wodurch sich eine Änderung dC5re = C5re,2 - C5re,1 ergibt,
(c33) einen C6+ Olefinproduktstrom, enthaltend Hexene und optional Olefine mit mehr als sechs C-Atomen, der mindestens teilweise mit einem Anteil C6+re zum OTO-Reaktor zurückgeführt wird, wobei der rückgeführte Anteil C6+re von einem ersten Wert C6+re,1 auf einen zweiten Wert C6+re,2 erhöht wird, wodurch sich eine Änderung dC6+re = C6+re,2 - C6+re,1 ergibt,
wobei sich die Änderung dC4+re als Summe der Änderungen dC4re + dC5re + dC6+re ergibt.

Vorteilhaft ist dabei die hiermit erhaltene hohe Flexibilität des Verfahrens, da mehrere Produktströme (C31), (c32), (c33) einzeln oder in Kombination zur Kompensation der Reduzierung des Anteils C2re des Ethylenproduktstroms, der zum OTO-Reaktor zurückgeführt wird, zur Verfügung stehen.

Ein siebter Aspekt der Erfindung ist dadurch gekennzeichnet, dass aus der Gruppe weiterer Olefinproduktströme nur der C4 Olefinproduktstrom mindestens teilweise mit einem Anteil C4re zum OTO-Reaktor zurückgeführt wird. Dies hat den Vorteil, dass somit ein im Vergleich zu anderen Olefinen weniger begehrtes Produkt, nämlich C4 Olefine, in höherem Maße zu den begehrten Olefinen Ethylen und Propylen umgesetzt werden.

Ein achter Aspekt der Erfindung ist dadurch gekennzeichnet, dass aus der Gruppe weiterer Olefinproduktströme nur der C5 Olefinproduktstrom mindestens teilweise mit einem Anteil C5re zum OTO-Reaktor zurückgeführt wird. Dies hat den Vorteil, dass sich aus stöchiometrischen Gründen durch die katalytische Spaltung der in verstärktem Maße zurückgeführten C5 Olefine sowohl die Ausbeute an Ethylen als auch die Ausbeute an Propylen verbessert.

Ein neunter Aspekt der Erfindung ist dadurch gekennzeichnet, dass aus der Gruppe weiterer Olefinproduktströme nur der C6+ Olefinproduktstrom mindestens teilweise mit einem Anteil C6+re zum OTO-Reaktor zurückgeführt wird. Dies hat den Vorteil, dass enthaltene C6+ Olefine, die im Vergleich zu kürzerkettigen Olefinen eine höhere Reaktivität für die katalytische Spaltung zu Ethylen und Propylen aufweisen, in verstärktem Maße für die Erzeugung dieser Zielprodukte genutzt werden können. Darüber hinaus enthält dieser Strom weitere reaktive Verbindungen, beispielsweise Cycloparaffine (gesättigte Naphthene) und Cycloolefine (ungesättigte Naphthene), die ebenfalls zu Ethylen und Propylen reagieren können, wie Untersuchungen gezeigt haben.

Ein zehnter Aspekt der Erfindung ist dadurch gekennzeichnet, dass aus der Gruppe weiterer Olefinproduktströme zwei dieser Ströme mindestens teilweise zum OTO-Reaktor zurückgeführt werden. In einem Beispiel wird der C4 Olefinproduktstrom und der C5 Olefinproduktstrom mindestens teilweise zum OTO-Reaktor zurückgeführt. In einem Beispiel wird der C4 Olefinproduktstrom und der C6+ Olefinproduktstrom mindestens teilweise zum OTO-Reaktor zurückgeführt. In einem Beispiel wird der C5 Olefinproduktstrom und der C6+ Olefinproduktstrom mindestens teilweise zum OTO-Reaktor zurückgeführt. Die Ausgestaltungen gemäß der beschriebenen Beispiele haben den Vorteil einer erhöhten Flexibilität der Verfahrensdurchführung.

Ein elfter Aspekt der Erfindung ist dadurch gekennzeichnet, dass alle weiteren Olefinproduktströme mindestens teilweise zum OTO-Reaktor zurückgeführt werden. Dies hat den Vorteil einer erhöhten Produktion der Zielprodukte Ethylen und Propylen, wohingegen sich der Energieaufwand für die Trennung bzw. Reinigung der weiteren Olefinproduktströme reduziert. Ferner werden die einzelnen Verfahrensstufen gleichmäßiger ausgelastet und ein neuer stationärer Zustand 2 schneller erreicht.

Ein zwölfter Aspekt der Erfindung ist dadurch gekennzeichnet, dass das Absenken des Anteils C2re und das Erhöhen mindestens eines Anteils, ausgewählt aus der Gruppe, umfassend: C4+re, C4re, C5re, C6+re,
so erfolgen, dass das Ethylen/Propylen-Produktverhältnis zwischen 1 und 20 Gew.-%, bevorzugt zwischen 2 und 20 Gew.-% beträgt. Untersuchungen zeigen, dass Ethylen/Propylen-Produktverhältnisse in dem genannten Wertebereich besonders gut und flexibel in einer Ethylen/Propylen-Copolymerisation zur Erzeugung von Polypropylen mit Ethylen als Co-Monomer verarbeitet werden können.

Ein dreizehnter Aspekt der Erfindung ist dadurch gekennzeichnet, dass das Absenken des Anteils C2re und das Erhöhen mindestens eines Anteils, ausgewählt aus der Gruppe, umfassend: C4+re, C4re, C5re, C6+re,
so erfolgen, dass ein stationärer Betrieb des Gesamtverfahrens und der einzelnen Verfahrensstufen gewährleistet ist. Dies verbessert die Verfügbarkeit des Verfahrens und es werden Stillstände und Notabschaltungen vermieden.

Ein vierzehnter Aspekt der Erfindung ist dadurch gekennzeichnet, dass das Verfahren das Bevorraten des Ethylens in einem Ethylen-Lagertank umfasst und dass das Absenken des Anteils C2re und das Erhöhen mindestens eines Anteils, ausgewählt aus der Gruppe, umfassend: C4+re, C4re, C5re, C6+re,
so erfolgen, dass ein zuvor festgelegtes Füllniveau des Ethylens im Ethylen-Lagertank nicht überschritten oder unterschritten wird. Hierdurch kann der Ethylen-Lagertank kleiner dimensioniert werden und es entfällt eine etwaige Überdimensionierung. Dies spart Aufstellplatz, Energie für Druckerzeugung und ggf. Kälteerzeugung und Kosten.

Ein fünfzehnter Aspekt der Erfindung ist dadurch gekennzeichnet, dass der Olefinanteil in den zurückgeführten, Olefine enthaltenden Stoffströmen mindestens 20 Gew.-%, bevorzugt mindestens 30 Gew.-%, meist bevorzugt mindestens 40 Gew.-% beträgt. Untersuchungen zeigen, dass diese Olefinanteile für unterschiedliche OTO-Verfahren und insbesondere für das MTP-Verfahren beobachtet werden und sich für die erfindungsgemäße Steigerung der Ethylenproduktion gut eignen.

Ein sechzehnter Aspekt der Erfindung ist dadurch gekennzeichnet, dass das Absenken des Anteils C2re und das Erhöhen mindestens eines Anteils, ausgewählt aus der Gruppe, umfassend: C4+re, C4re, C5re, C6+re, nachfolgend rückgängig gemacht werden, um das Verfahren von einem zweiten Zustand 2 mit höherer Ethylenproduktion zurück in einen ersten Zustand 1 mit niedrigerer Ethylenproduktion zu überführen. Dies erhöht die Flexibilität des Verfahrens, falls beispielsweise in einer nachgeschalteten Polymerisationsanlage eine Produktionskampagne zur Erzeugung von Polypropylen mit geringem Anteil an Ethylen als Co-Monomer durchgeführt werden soll.

Ein siebzehnter Aspekt der Erfindung ist dadurch gekennzeichnet, dass das Verfahren das Bevorraten des Ethylens in einem Ethylen-Lagertank umfasst und dass das Überführen des Verfahrens in die Zustände 1 oder 2 mit der Bedingung erfolgt, dass ein zuvor festgelegtes Füllniveau des Ethylens im Ethylen-Lagertank nicht überschritten oder unterschritten wird. Hierdurch kann der Ethylen-Lagertank kleiner dimensioniert werden und es entfällt eine etwaige Überdimensionierung. Dies spart Aufstellplatz, Energie für Druckerzeugung und ggf. Kälteerzeugung und Kosten.

Ein achtzehnter Aspekt der Erfindung ist dadurch gekennzeichnet, dass der zweite Wert C2re,2 Null ist, so dass der Ethylen enthaltende Ethylenproduktstrom vollständig aus dem Verfahren ausgeleitet wird. Dies maximiert die Ethylen-Ausbeute, wohingegen sich die Ausbeute an Propylen nur geringfügig oder gar nicht ändert.

### Ausführungsbeispiele

Weitere Merkmale, Vorteile und Anwendungsmöglichkeiten der Erfindung ergeben sich aus der nachfolgenden Beschreibung von Ausführungs- und Zahlenbeispielen und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbezügen.

Es zeigt die einzige Figur:

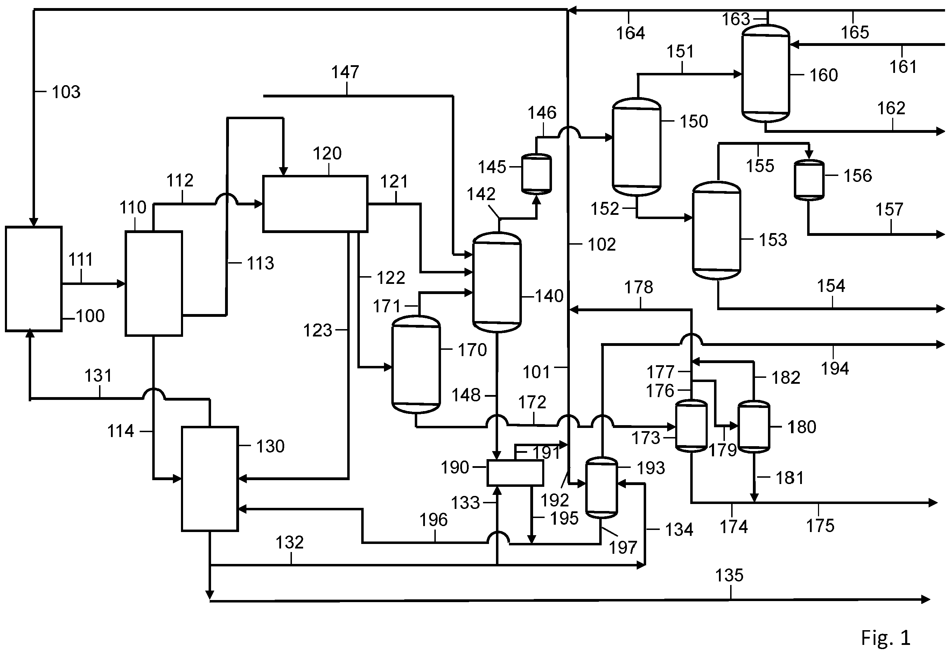

- Fig. 1: eine schematische Darstellung eines Verfahrens gemäß der Erfindung.

Figur 1 zeigt als Sonderfall eines OTO-Verfahrens das Fließschema einer MTP-Anlage zur Durchführung der Erfindung, die sich zunächst im Zustand 1 befindet.

Der OTO-Reaktor 100, in dem die Bildung von Olefinen aus Oxygenaten wie beispielsweise Methanol und/oder Dimethylether (DME) abläuft, wird über eine nicht gezeigte Frischfeed-Leitung mit Oxygenaten sowie Wasserdampf als Verdünnungsmittel beaufschlagt; ferner werden über Leitungen 103 und 131 auch Rückführströme zum Reaktor zurückgeführt. Über Leitung 111 wird der Produktstrom des Reaktors 100 in das Quenchsystem 110 eingebracht. Eine im wesentlichen Wasser enthaltene Phase wird über Leitung 114 aus dem Quenchsystem 110 abgeführt und in eine Methanolrückgewinnungseinheit 130 eingebracht. Die im Quenchsystem 110 abgetrennte gasförmige Phase gelangt über Leitung 112 in einen Kompressor 120. Über Leitung 113 wird zudem eine flüssige, im Wesentlichen aus Kohlenwasserstoffen bestehende Phase aus dem Quenchsystem 110 in den Kompressor 120 eingespeist.

Aus dem Kompressor 120 wird ein flüssiger Strom über Leitung 121 in eine Trennvorrichtung 140 geführt. In dieser Trennvorrichtung 140 wird die C3- Fraktion von der C4 Fraktion getrennt, weshalb sie auch als Depropanizer bezeichnet wird. Die Trennvorrichtung 140 ist vorzugsweise als Trennkolonne ausgestaltet. Vorzugsweise erfolgt die Rektifikation in der Trennvorrichtung 140 als Extraktivdestillation, weshalb über Leitung 147 ein Extraktionsmittel, beispielsweise Methanol, zugeführt werden kann, soweit dies gewünscht ist.

Das über Kopf abgezogene Kopfprodukt, welches die C3- Fraktion enthält, wird über Leitung 142 einem Trockner 145 zugeführt, von dem aus es weiter über Leitung 146 in eine weitere Trennvorrichtung 150 gelangt. Auch eine direkte Verbindung von Trennvorrichtung 140 mit Trennvorrichtung 150 ist möglich. In dieser Trennvorrichtung 150 wird die C3 Fraktion von der C2- Fraktion getrennt, weshalb die Trennvorrichtung 150 auch als Deethanizer bezeichnet wird.

Über Leitung 151 gelangt das Kopfprodukt der Trennvorrichtung 150 in eine Trennvorrichtung 160, welche vorzugsweise als Wäscher ausgebildet ist. Bei einer Ausbildung als Wäscher wird über Leitung 161 ein Waschmittel, beispielsweise Waschwasser eingebracht und über Leitung 162 wieder abgezogen. Der enthaltene C2- Strom wird mit Leitung 163 über Kopf abgezogen und kann über Leitung 164 einem zurück in den Reaktor 100 führenden Recyclestrom in Leitung 103 zugeführt werden und/oder über Leitung 165 aus dem System ausgeleitet und einer weiteren, bildlich nicht gezeigten Trennvorrichtung zur Aufreinigung der enthaltenen Produkte zugeführt werden, um beispielsweise enthaltenes Ethylen als Reinprodukt zu gewinnen.

Das Sumpfprodukt der Trennvorrichtung 150 wird über Leitung 152 einer Trennvorrichtung 153 zugeführt, welche Propan von Propylen trennt. In diesem sogenannten C3 Splitter wird über Leitung 154 Propan aus dem System abgezogen, welches z. B. als Brenngas Verwendung finden kann. Über Leitung 155 wird das Zielprodukt Propylen abgezogen, welches optional in Abhängigkeit von Qualitätsanforderungen einer weiteren Aufreinigungsvorrichtung 156 zugeführt werden kann, aus der es dann über Leitung 157 ausgeleitet wird. Die Aufreinigungsvorrichtung 156 kann beispielsweise mit einem für Oxygenate selektiven Sorbens befüllt werden, so dass Oxygenate aus dem Propylen-Produktstrom je nach Spezifikation bis auf geringe Spuren entfernt werden können.

Über Leitung 122 wird aus dem Kompressor eine flüssige Phase abgezogen, welche einer Trennvorrichtung 170 zugeführt wird, in der die C4- Fraktion und die Oxygenate von der C4+ Fraktion getrennt werden. Diese Trennvorrichtung 170 wird auch als Debutanizer bezeichnet. Über Leitung 171 gelangt die C4- Fraktion und das Oxygenat enthaltende Kopfprodukt der Trennvorrichtung 170 in die Trennvorrichtung 140.

Über Leitung 172 wird das Sumpfprodukt der Trennvorrichtung 170 in eine weitere Trennvorrichtung 173 gegeben, in der die C7+ Fraktion von der C6 Fraktion abgetrennt wird, weshalb die Trennvorrichtung 173 auch als Dehexanizer bezeichnet wird. Das Sumpfprodukt wird über Leitung 174, 175 abgezogen. Über Leitung 176, 177 und 178 kann ein Recyclestrom einer Leitung 101 zugeführt werden, die über Leitungen 102 und 103 zurück zum Reaktor 100 führt.

Aus Leitung 176 kann überdies eine Leitung 179 abgezweigt werden, welche in eine Trennvorrichtung 180 (Benzin-Stabilisatorkolonne) geführt werden. Das hier erhaltene Benzin wird als Wertprodukt über Leitung 181 und Leitung 175 ausgeschleust. Die über Kopf abgetrennten C4 Kohlenwasserstoffe werden über Leitung 182 den Leitungen 177 und 178 zugeführt, so dass sie letztendlich in den Recyclestrom in Leitungen 102, 103 münden.

Das Sumpfprodukt der Trennvorrichtung 140 wird über Leitung 148 abgezogen und gelangt so in eine Mixer-Settler-Kombination 190. Aus dieser werden Kohlenwasserstoffe über Leitung 191 abgezogen und können vollständig oder teilweise über Leitungen 101, 102, 103 in den Reaktor 100 zurückgeführt werden. Alternativ oder ergänzend ist es möglich, die Kohlenwasserstoffe über Leitung 192 einer Trennvorrichtung 193 zuzuführen, welche vorzugsweise als Extraktion ausgestaltet ist. Das Kopfprodukt aus der Trennvorrichtung 193 wird über Leitung 194 als Brenngas ausgeschleust.

Aus der Methanol-Dimethylether-Rückgewinnung 130 wird zum einen ein Oxygenat-Recyclestrom über Leitung 131 in den Reaktor 100 zurückgeführt. Über Leitungen 132 und 133 kann optional mit Methanol und/oder mit Dimethylether beladenes Wasser in die Mixer-Settler-Einheit 190 gespeist werden, aus der es dann auch über die Leitung 195 wieder der Leitung 197 und 196 zugeführt werden kann.

Über Leitungen 132, 134 kann das Wasser als Extraktionsmittel in die Trennvorrichtung 193 geführt werden, sofern diese als Extraktion ausgestaltet ist, aus der es dann auch über die Leitung 197 wieder der Leitung 196 zugeführt werden kann. Über Leitung 135 kann schließlich das Wasser aus dem System ausgeleitet werden.

Erfindungsgemäß erfolgt die Umstellung des Verfahrens ausgehend von einem Zustand 1 mit niedrigerer Ethylenproduktion hin zu einem Zustand 2 mit höherer Ethylenproduktion dadurch, dass folgende Änderungen vorgenommen werden:
(1) Der Anteil C2re des Ethylenproduktstroms, der zum OTO-Reaktor zurückgeführt wird, wird von einem ersten Wert C2re,1 auf einen zweiten Wert C2re,2 abgesenkt, wodurch sich eine Änderung dC2re = C2re,1 - C2re,2 ergibt. In einem Beispiel wird der Anteil C2re des Ethylenproduktstroms auf einen zweiten Wert C2re,2 von Null abgesenkt, so dass der Ethylen enthaltende Ethylenproduktstrom vollständig aus dem Verfahren ausgeleitet wird.
   Dies wird im Beispiel der Fig. 1 beispielsweise realisiert durch teilweises oder vollständiges Schließen eines im Leitungsweg der Leitung 164 befindliches, bildlich nicht gezeigtes Dosier- oder Absperrventil.
(2) Der Anteil C4+re des C4+ Olefine enthaltenden C4+ Produktstroms, der zum OTO-Reaktor zurückgeführt wird, wird von einem ersten Wert C4+re,1 auf einen zweiten Wert C4+re,2 erhöht, wodurch sich eine Änderung dC4+re = C4+re,2 - C4+re,1 ergibt.

Dies wird im Beispiel der Fig. 1 beispielsweise realisiert durch Erhöhen der in der Leitungen 176 und 182 zurückgeführten Stoffmengenströme mittels in Fig. 1 bildlich nicht gezeigter Dosiervorrichtungen.

Dabei stehen die Änderungen dC2re und dC4+re in einem Massenstromverhältnis dC4+re / dC2re, wobei das Massenstromverhältnis zwischen 70 % und 130 %, bevorzugt zwischen 80 % und 120 %, weiter bevorzugt zwischen 90 % und 110 %, meist bevorzugt 100% beträgt.

Um eine Polymerisation zu verhindern, kann in einem Beispiel ein Inhibitor zu den Kondensatoren und Aufkochern wenigstens einer Trennkolonne, insbesondere dem Debutanizer, Dehexanizer und einer ggf. vorhandenen Benzin-Stabilisatorkolonne hinzugefügt werden.

### Zahlenbeispiele

Das in Fig. 1 dargestellte MTP-Verfahren wurde zunächst im Zustand 1 betrieben. Die Selektivität für Ethylen + Propylen betrug dabei 67 %, verteilt auf 65 % für Propylen und 2 % für Ethylen.

Anschließend wurde das MTP-Verfahren durch die erfindungsgemäßen Maßnahmen in den Zustand 2 überführt, wobei der zum OTO-Reaktor zurückgeführte Anteil C2re auf Null abgesenkt wurde. Die Selektivität für Ethylen + Propylen betrug im Zustand 2 sodann 76 %, verteilt auf 65 % für Propylen und 11 % für Ethylen.

Somit ist ersichtlich, dass die hohe Propylen-Selektivität durch die erfindungsgemäße Änderung nicht nachteilig beeinflusst wurde. Dagegen stieg die Ethylen-Selektivität um 9 %. Es reduzierten sich proportional die über die Leitungen 154, 175 bzw. 194 ausgeleiteten Massenströme.

In den nachfolgenden Tabellen sind Betriebsdaten für das erfindungsgemäße Verfahren für die Betriebszustände "start of run" (SOR), Betriebsbeginn, Betriebsdauer unter 1000 hos, und "middle of run" (MOR), mittlerer Betriebsabschnitt, Betriebsdauer über 1000 hos, zusammengestellt.

Wie anhand der in den Tabellen zusammengestellten Daten ersichtlich ist, steigt im Betriebszeitraum Start of Run (SOR) beim Übergang von Zustand 1 nach Zustand 2 die Ethylenproduktion um 166 t/h, während die Propylenproduktion zunächst leicht um 36 t/d abnimmt.

Dagegen steigt im Betriebszeitraum Middle of Run (MOR) beim Übergang von Zustand 1 nach Zustand 2 die Ethylenproduktion um 171 t/h, während die Propylenproduktion konstant bleibt.

Da der Verfahrensbetrieb in den Beispielen mit produktionsfrischem OTO-Katalysator begonnen wurde, erfolgte während des Betriebszeitraums "Start of Run" (SOR) auch die Formierung oder Konditionierung des OTO-Katalysators, die zunächst zu einem konstanten Reaktionsverhalten des OTO-Katalysators führt ("Middle of Run", MOR), bevor - bei sehr langen Betriebszeiten im Betriebszeitraum "End of Run" (EOR) bei typischerweise mehr als 7000 hos - Alterungseffekte auftreten. Es wurde beobachtet, dass sich die Formierungsphase oder Konditionierungsphase verkürzt, wenn das Verfahren frühzeitig im Betriebszeitraum "Start of Run (SOR)" von Zustand 1 nach Zustand 2 überführt wird. In einem Beispiel erfolgt das Überführen des Verfahrens von einem ersten Zustand 1 mit niedrigerer Ethylenproduktion in einen zweiten Zustand 2 mit höherer Ethylenproduktion spätestens nach 200 Betriebsstunden. In einem weiteren Beispiel erfolgt das Überführen des Verfahrens von einem ersten Zustand 1 mit niedrigerer Ethylenproduktion in einen zweiten Zustand 2 mit höherer Ethylenproduktion spätestens nach 100 Betriebsstunden. In einem weiteren Beispiel erfolgt das Überführen des Verfahrens von einem ersten Zustand 1 mit niedrigerer Ethylenproduktion in einen zweiten Zustand 2 mit höherer Ethylenproduktion spätestens nach 50 Betriebsstunden.

**Tabelle 1: Betriebsparameter für Middle of Run (MOR) für 5000 t/d Methanol als Einsatzstrom, Betriebsdauer mehr als 1000 hos (1000 bis 7000 hos, Mittelwerte)**

| **Parameter** | **Vergleichsbeisp., mit C2 Recycle (Zustand 1)** | **Erfindung, ohne C2 Recycle (Zustand 2)** | **Differenz** |
|---|---|---|---|
| Ethylenproduktion (t/d) | 60 | 231 | 171 |
| Propylen produktion (t/d) | 1420 | 1420 | 0 |
| LPG-Produktion C3 + C4 + C4= (t/d) | 110 | 67 | |
| Benzinproduktion C5+ (t/d) | 540 | 420 | |
| C2- Paraffine (t/d) | 57 | 49 | |
| Kohlenwasserstoffe gesamt (t/d) | 2187 | 2187 | |
| C2 Recycle (t/h) | 26, davon : | 0 | -26 |
| Leitung 164 | Olefine: 20 | | -20 |
| | Paraffine: 6 | | |
| C4 Recycle (t/h) | 40,davon : | 50 | +10 |
| Leitung 191 | Olefine: 16,4 | Olefine: 17,8 | +1,4 |
| | Paraffine: 23,6 | Paraffine: 32,2 | |
| C5/C6/C7 Recycle (t/h), | 140, davon : | 156 | +16 |
| Leitung 178 | Olefine: 53,4 | Olefine: 59,3 | +5,9 |
| | Paraffine: 86,6 | Paraffine:96,7 | |
| Olefine gesamt im Recycle (t/h) | 89,8 | 77,1 | -12,7 |
| Gesamt-Recycle (t/h) Leitung 103 | 206 | 206 | 0 |

**Tabelle 2: Betriebsparameter für Start of Run (SOR) für 5000 t/d Methanol als Einsatzstrom, Betriebsdauer Null bis 1000 h (Mittelwerte)**

| **Parameter** | **Vergleichsbeisp., mit C2 Recycle (Zustand 1)** | **Erfindung, ohne C2 Recycle (Zustand 2)** | **Differenz** |
|---|---|---|---|
| Ethylenproduktion (t/d) | 60 | 226 | 166 |
| Propylen produktion (t/d) | 1340 | 1304 | -36 |
| LPG-Produktion | 125 | 100 | |
| C3 + C4 + C4= (t/d) | | | |
| Benzinproduktion C5+ (t/d) | 600 | 505 | |
| C2- Paraffine (t/d) | 62 | 52 | |
| Kohlenwasserstoffe gesamt (t/d) | 2187 | 2187 | |
| C2 Recycle (t/h) | 26, davon : | 0 | -26 |
| Leitung 164 | Olefine: 16 | | -16 |
| | Paraffine: 10 | | |
| C4 Recycle (t/h) | 40,davon : | 50 | +10 |
| Leitung 191 | Olefine: 12 | Olefine: 16 | +4 |
| | Paraffine: 28 | Paraffine: 34 | |
| C5/C6/C7 Recycle (t/h), | 140, davon : | 156 | +16 |
| Leitung 178 | Olefine: 45 | Olefine: 52 | +7 |
| | Paraffine: 95 | Paraffine: 104 | |
| Olefine gesamt im Recycle (t/h) | 73 | 68 | -5 |
| Gesamt-Recycle (t/h) | 206 | 206 | 0 |
| Leitung 103 | | | |

### Bezugszeichenliste

- 100: OTO-Reaktor
- 101-103: Leitung
- 110: Quench-System
- 111-114: Leitung
- 120: Kompressor
- 121-123: Leitung
- 130: Methanol-DME-Rückgewinnung
- 131-135: Leitung
- 140: Trennvorrichtung
- 142: Leitung
- 150: Trennvorrichtung
- 151, 152: Leitung
- 153: Trennvorrichtung
- 154, 155: Leitung
- 156: Aufreinigungsstufe
- 157: Leitung
- 160: Trennvorrichtung
- 161-165: Leitung
- 170: Trennvorrichtung
- 171, 172: Leitung
- 173: Trennvorrichtung
- 174-179: Leitung
- 180: Trennvorrichtung (Benzin-Stabilisatorkolonne)
- 181, 182: Leitung
- 190: Mixer-Settler-Kombination
- 191,192: Leitung
- 193: Trennvorrichtung
- 194-197: Leitung
- 200: Reaktor
- 210: Quench-System
- 211-214: Leitung
- 220: Kompressor
- 221-223: Leitung
- 230: Methanol-DME-Rückgewinnung
- 231-235: Leitung
- 240: Trennvorrichtung
- 242: Leitung
- 250: Trennvorrichtung
- 251, 252: Leitung
- 253: Trennvorrichtung
- 254, 255: Leitung
- 256: Aufreinigungsstufe
- 257: Leitung
- 260: Trennvorrichtung
- 261-265: Leitung
- 270: Trennvorrichtung
- 271: Leitung
- 280: Trennvorrichtung (Benzin-Stabilisatorkolonne)
- 281, 282: Leitung
- 290: Mixer-Settler-Kombination
- 291, 292: Leitung
- 293: Trennvorrichtung
- 294-297: Leitung
- 300-306: Leitung
- 310: Verdampfer
- 311, 312: Leitung
- 320-323: Leitung

## Patentansprüche

1. Verfahren zur Herstellung von Olefinen aus Oxygenaten mit variabler Produktion an Ethylen und Propylen, umfassend die folgenden Schritte:
(a) Bereitstellen eines Oxygenate enthaltenden Einsatzstromes und heterogen-katalysiertes Umsetzen der Oxygenate in dem Einsatzstrom in einem Oxygenat-zu-Olefin-Reaktor (OTO-Reaktor), enthaltend einen Oxygenat-zu-Olefin-Katalysator (OTO-Katalysator), unter Oxygenatumwandlungsbedingungen zu einem Reaktorproduktstrom, der Wasser, Kohlenwasserstoffe und wenigstens ein Oxygenat enthält,
(b) Quenchen des Reaktorproduktstroms in wenigstens einer Quenchstufe, wodurch folgende Stoffströme erhalten werden:
(b1) ein gasförmiger Strom G, der Kohlenwasserstoffe umfasst und der spezifisch Olefine umfasst,
(b2) ein flüssiger Strom W, der Wasser und Oxygenate umfasst,
(b3) ein flüssiger Strom O, der Kohlenwasserstoffe umfasst und der spezifisch Olefine umfasst,
(c) Mehrstufiges Auftrennen des Stroms G und/oder des Stroms O mittels thermischer Trennverfahren, wobei eine Gruppe von Produktströmen erhalten wird, wobei die Gruppe von Produktströmen umfasst:
(c1) einen Ethylen enthaltenden Ethylenproduktstrom, der mindestens teilweise aus dem Verfahren ausgeleitet wird, wobei ein weiterer Anteil C2re des Ethylenproduktstroms zum OTO-Reaktor zurückgeführt wird,
(c2) einen Propylen enthaltenden Propylenproduktstrom, der aus dem aus dem Verfahren ausgeleitet wird,
(c3) mindestens einen C4+ Olefine enthaltenden C4+ Produktstrom, der mindestens teilweise mit einem Anteil C4+re zum OTO-Reaktor zurückgeführt wird,
(c4) einen Benzinproduktstrom, der C4+ Paraffine und Aromaten umfasst und aus dem Verfahren ausgeleitet wird, wobei
(d) das Verfahren von einem ersten Zustand 1 mit niedrigerer Ethylenproduktion in einen zweiten Zustand 2 mit höherer Ethylenproduktion überführt wird durch: (e) Absenken des Anteils C2re von einem ersten Wert C2re,1 auf einen zweiten Wert C2re,2, wodurch sich eine Änderung dC2re = C2re,1 - C2re,2 ergibt,
(e) Erhöhen des Anteils C4+re von einem ersten Wert C4+re,1 auf einen zweiten Wert C4+re,2, wodurch sich eine Änderung dC4+re = C4+re,2 - C4+re,1 ergibt,
(f) wobei die Änderungen dC2re und dC4+re in einem Massenstromverhältnis dC4+re / dC2re stehen, wobei das Massenstromverhältnis zwischen 70 % und 130 %, bevorzugt zwischen 80 % und 120 %, weiter bevorzugt zwischen 90 % und 110 %, meist bevorzugt 100% beträgt.

2. Verfahren nach Anspruch 1, **dadurch gekennzeichnet, dass** sich beim Überführen des Verfahrens von einem ersten Zustand 1 mit niedrigerer Ethylenproduktion in einen zweiten Zustand 2 mit höherer Ethylenproduktion die Propylenproduktion sich nur um maximal 5 %, bevorzugt nur um maximal 3 % verringert.

3. Verfahren nach Anspruch 1 oder 2, **dadurch gekennzeichnet, dass** sich beim Überführen des Verfahrens von einem ersten Zustand 1 mit niedrigerer Ethylenproduktion in einen zweiten Zustand 2 mit höherer Ethylenproduktion die Summe (Ethylenproduktion + Propylenproduktion) um mindestens 8 %, bevorzugt um mindestens 10 % erhöht.

4. Verfahren nach einem der vorigen Ansprüche, **dadurch gekennzeichnet, dass** das Überführen des Verfahrens von einem ersten Zustand 1 mit niedrigerer Ethylenproduktion in einen zweiten Zustand 2 mit höherer Ethylenproduktion frühestens nach 600 Betriebsstunden, bevorzugt frühestens nach 800 Betriebsstunden, meist bevorzugt frühestens nach 1000 Betriebsstunden des OTO-Katalysators erfolgt.

5. Verfahren nach einem der vorigen Ansprüche, **dadurch gekennzeichnet, dass** das Überführen des Verfahrens von einem ersten Zustand 1 mit niedrigerer Ethylenproduktion in einen zweiten Zustand 2 mit höherer Ethylenproduktion spätestens nach 200 Betriebsstunden, bevorzugt spätestens nach 100 Betriebsstunden, meist bevorzugt spätestens nach 50 Betriebsstunden des OTO-Katalysators erfolgt.

6. Verfahren nach einem der vorigen Ansprüche, **dadurch gekennzeichnet, dass** der C4+ Olefine enthaltende C4+ Produktstrom mittels thermischer Trennverfahren weiter aufgetrennt wird in mindestens einen weiteren Olefinproduktstrom, ausgewählt aus einer Gruppe, die umfasst:
(c31) einen C4 Olefinproduktstrom, enthaltend Butene, der mindestens teilweise mit einem Anteil C4re zum OTO-Reaktor zurückgeführt wird, wobei der rückgeführte Anteil C4re von einem ersten Wert C4re,1 auf einen zweiten Wert C4re,2 erhöht wird, wodurch sich eine Änderung dC4re = C4re,2 - C4re,1 ergibt,
(c32) einen C5 Olefinproduktstrom, enthaltend Pentene, der mindestens teilweise mit einem Anteil C5re zum OTO-Reaktor zurückgeführt wird, wobei der rückgeführte Anteil C5re von einem ersten Wert C5re,1 auf einen zweiten Wert C5re,2 erhöht wird, wodurch sich eine Änderung dC5re = C5re,2 - C5re,1 ergibt,
(c33) einen C6+ Olefinproduktstrom, enthaltend Hexene und optional Olefine mit mehr als sechs C-Atomen, der mindestens teilweise mit einem Anteil C6+re zum OTO-Reaktor zurückgeführt wird, wobei der rückgeführte Anteil C6+re von einem ersten Wert C6+re,1 auf einen zweiten Wert C6+re,2 erhöht wird, wodurch sich eine Änderung dC6+re = C6+re,2 - C6+re,1 ergibt,
wobei sich die Änderung dC4+re als Summe der Änderungen dC4re + dC5re + dC6+re ergibt.

7. Verfahren nach einem der vorigen Ansprüche , **dadurch gekennzeichnet, dass** aus der Gruppe weiterer Olefinproduktströme nur der C4 Olefinproduktstrom mindestens teilweise mit einem Anteil C4re zum OTO-Reaktor zurückgeführt wird.

8. Verfahren nach einem der vorigen Ansprüche, **dadurch gekennzeichnet, dass** aus der Gruppe weiterer Olefinproduktströme nur der C5 Olefinproduktstrom mindestens teilweise mit einem Anteil C5re zum OTO-Reaktor zurückgeführt wird.

9. Verfahren nach einem der vorigen Ansprüche , **dadurch gekennzeichnet, dass** aus der Gruppe weiterer Olefinproduktströme nur der C6+ Olefinproduktstrom mindestens teilweise mit einem Anteil C6+re zum OTO-Reaktor zurückgeführt wird.

10. Verfahren nach einem der vorigen Ansprüche , **dadurch gekennzeichnet, dass** aus der Gruppe weiterer Olefinproduktströme zwei dieser Ströme mindestens teilweise zum OTO-Reaktor zurückgeführt werden.

11. Verfahren nach einem der vorigen Ansprüche , **dadurch gekennzeichnet, dass** alle weiteren Olefinproduktströme mindestens teilweise zum OTO-Reaktor zurückgeführt werden.

12. Verfahren nach einem der vorigen Ansprüche, **dadurch gekennzeichnet, dass** das Absenken des Anteils C2re und das Erhöhen mindestens eines Anteils, ausgewählt aus der Gruppe, umfassend: C4+re, C4re, C5re, C6+re,
so erfolgen, dass das Ethylen/Propylen-Produktverhältnis zwischen 1 und 20 Gew.-%, bevorzugt zwischen 2 und 20 Gew.-% beträgt.

13. Verfahren nach einem der vorigen Ansprüche, **dadurch gekennzeichnet, dass** das Absenken des Anteils C2re und das Erhöhen mindestens eines Anteils, ausgewählt aus der Gruppe, umfassend: C4+re, C4re, C5re, C6+re,
so erfolgen, dass ein stationärer Betrieb des Gesamtverfahrens und der einzelnen Verfahrensstufen gewährleistet ist.

14. Verfahren nach einem der vorigen Ansprüche, **dadurch gekennzeichnet, dass** das Verfahren das Bevorraten des Ethylens in einem Ethylen-Lagertank umfasst und dass das Absenken des Anteils C2re und das Erhöhen mindestens eines Anteils, ausgewählt aus der Gruppe, umfassend: C4+re, C4re, C5re, C6+re, so erfolgen, dass ein zuvor festgelegtes Füllniveau des Ethylens im Ethylen-Lagertank nicht überschritten oder unterschritten wird.

15. Verfahren nach einem der vorigen Ansprüche, **dadurch gekennzeichnet, dass** der Olefinanteil in den zurückgeführten, Olefine enthaltenden Stoffströmen mindestens 20 Gew.-%, bevorzugt mindestens 30 Gew.-%, meist bevorzugt mindestens 40 Gew.-% beträgt.

16. Verfahren nach Anspruch 1 oder 2, **dadurch gekennzeichnet, dass** das Absenken des Anteils C2re und das Erhöhen mindestens eines Anteils, ausgewählt aus der Gruppe, umfassend: C4+re, C4re, C5re, C6+re, nachfolgend rückgängig gemacht werden, um das Verfahren von einem zweiten Zustand 2 mit höherer Ethylenproduktion zurück in einen ersten Zustand 1 mit niedrigerer Ethylenproduktion zu überführen.

17. Verfahren nach Anspruch 12, **dadurch gekennzeichnet, dass** das Verfahren das Bevorraten des Ethylens in einem Ethylen-Lagertank umfasst und dass das Überführen des Verfahrens in die Zustände 1 oder 2 mit der Bedingung erfolgt, dass ein zuvor festgelegtes Füllniveau des Ethylens im Ethylen-Lagertank nicht überschritten oder unterschritten wird.

18. Verfahren nach einem der vorigen Ansprüche, **dadurch gekennzeichnet, dass** der zweite Wert C2re,2 Null ist, so dass der Ethylen enthaltende Ethylenproduktstrom vollständig aus dem Verfahren ausgeleitet wird.
